# EUROPEAN PATENT APPLICATION

(11) **EP 4 736 933 A1**
(43) Date of publication of application: **06.05.2026**
(21) Application number: 25211249.5
(22) Date of filing: 27.10.2025
(51) Int. Cl.: A61N 1/05, A61N 1/372, A61N 1/375

(54) **BIOSTIMULATOR HAVING A RADIOPAQUE MARKER**

(30) Priority: 29.10.2024 US 202463713472 P; 23.10.2025 US 202519367692
(71) Applicant: Pacesetter, Inc., Sylmar, CA 91342 (US)
(72) Inventor: Teague, Bryan, Sylmar, CA 91342 (US); Welch, Mark, Sylmar, CA 91342 (US); Kwon, Daniel, Sylmar, CA 91342 (US); Chantasirivisal, Steve, Sylmar, CA 91342 (US)
(74) Representative: Barker Brettell Sweden AB

(57) **Abstract**

A biostimulator (100) includes a housing (302) having a longitudinal axis (304) and containing an electronics compartment (306). A pacing element (108) is coupled to the housing (302). The pacing element (108) includes a flexible conductor (410) extending along the longitudinal axis (304). A fixation element mount (311) is mounted on the housing (302). The fixation element mount (311) includes a distal mount end (430). A fixation element (106) is mounted on the fixation element mount (311). The fixation element (106) extends about the longitudinal axis (304). A radiopaque marker (502) has a distal marker end (504) longitudinally aligned with the distal mount end (430) of the fixation element mount (311). Other embodiments are also described and claimed.

## Description

### TECHNICAL FIELD

The present disclosure relates to biostimulators. More specifically, the present disclosure relates to leadless biostimulators useful for deep septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Deep septal pacing is an alternative to His-bundle pacing. Deep septal pacing involves pacing past the His-bundle toward the right ventricle apex. More particularly, deep septal pacing targets the left bundle branch below the His site. Deep septal pacing has been achieved using a lead in which the electrode penetrates several millimeters into the septum. Pacing thresholds associated with deep septal pacing are potentially lower than with His-bundle pacing, and clinical efficacy of the approach has been demonstrated.

### SUMMARY

Deep septal pacing, e.g., left bundle branch area pacing (LBBAP) can require a pacing electrode to penetrate through a majority of a ventricular septal wall to extend into the left bundle branch or into a left bundle fascicular that resides on a left side of a septum. In the case of a leadless cardiac pacemaker, a body of the pacemaker can be affixed to the septum and the pacing electrode can penetrate to the target tissue. More particularly, the pacing electrode may be required to penetrate 10-12 mm into the ventricular septal wall. Affixation of the leadless cardiac pacemaker to the septal wall and a depth of the pacing electrode should be accurately performed for effective treatment.

Existing leadless cardiac pacemakers are not well suited to ensuring affixation of the leadless cardiac pacemaker to the septal wall. Fluoroscopy may be used to visualize placement of the leadless cardiac pacemaker. A relative location between a fixation element and the septum may not be visible under such fluoroscopy, however. As a result, the leadless cardiac pacemaker may be over-torqued during implantation, which can lead to burrowing into the septal tissue too far and possible perforation of the septum, or under-torqued during implantation, which can lead to inadequate fixation of the leadless cardiac pacemaker to the septal wall. Accordingly, a form of feedback or indication under fluoroscopy to allow a physician to confirm that the leadless cardiac pacemaker is accurately affixed to the septal wall can be beneficial, e.g., by reducing a risk of dislodgement of the leadless cardiac pacemaker after implantation.

Existing leadless cardiac pacemakers employ fixation elements that may not adequately anchor the leadless cardiac pacemaker in the septum. Insufficient capture of septal tissue can increase a risk of the leadless cardiac pacemaker becoming dislodged after implantation. Accordingly, a fixation element that maximizes tissue capture can mitigate a risk of dislodgment.

Existing leadless cardiac pacemakers are not well suited to ensuring that a depth of the pacing electrode is accurate. A pacing electrode used to pace target tissue is commonly physically separated from a fixation element used to anchor the leadless cardiac pacemaker in the septum. The physical separation can make it difficult to burrow the electrically active electrode into the septum far enough to contact the left bundle branch without perforating into the left ventricle. Shallow burrowing, however, risks having poor anchoring in the septal tissue. Accordingly, a fixation element that can anchor the leadless cardiac pacemaker in the septal wall and deliver pacing impulses to the target tissue at an accurate depth can be beneficial.

A biostimulator is described. In an embodiment, the biostimulator includes a housing having a longitudinal axis and containing an electronics compartment. The biostimulator includes a pacing element coupled to the housing. The pacing element includes a flexible conductor extending along the longitudinal axis. The biostimulator includes a fixation element mount mounted on the housing. The fixation element mount includes a distal mount end. The biostimulator includes a fixation element mounted on the fixation element mount. The fixation element extends about the longitudinal axis. The biostimulator includes a radiopaque marker having a distal marker end longitudinally aligned with the distal mount end of the fixation element mount.

A biostimulator is described. In an embodiment, the biostimulator includes a housing having a longitudinal axis and containing an electronics compartment. The biostimulator includes a fixation element mount mounted on the housing. The biostimulator includes a fixation element mounted on the fixation element mount. The fixation element extends helically about the longitudinal axis. The fixation element includes a flared section having an outer diameter that increases in a distal direction.

A biostimulator is described. In an embodiment, the biostimulator includes a housing having a longitudinal axis and containing circuitry in an electronics compartment. The biostimulator includes a fixation element mount mounted on the housing. The biostimulator includes a fixation element mounted on the fixation element mount. The fixation element is electrically coupled to the circuitry. The fixation element includes an insulative coating between a proximal exposed section and a distal exposed section.

A biostimulator system is described. In an embodiment, the biostimulator system includes a biostimulator transport system, and any of the biostimulators referred to above mounted on the biostimulator transport system.

The above summary does not include an exhaustive list of all aspects of the present invention. It is contemplated that the invention includes all systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a perspective view of a biostimulator system, in accordance with an embodiment.
FIG. 3 is a side view of a biostimulator having a pacing element, in accordance with an embodiment.
FIG. 4 is a side view of a distal portion of a biostimulator having a pacing element, in accordance with an embodiment.
FIG. 5 is a side view of a distal portion of a biostimulator having a radiopaque marker, in accordance with an embodiment.
FIG. 6 is a sectional view of a distal portion of a biostimulator having a radiopaque marker, in accordance with an embodiment.
FIG. 7 is a sectional view of a distal portion of a biostimulator having a radiopaque marker, in accordance with an embodiment.
FIG. 8 is a sectional view of a distal portion of a biostimulator having a radiopaque marker, in accordance with an embodiment.
FIG. 9 is a sectional view of a distal portion of a biostimulator having a radiopaque marker, in accordance with an embodiment.
FIG. 10 is a side view of a tip electrode of a biostimulator, in accordance with an embodiment.
FIG. 11 is a side view of a distal portion of a biostimulator having a flared fixation element, in accordance with an embodiment.
FIG. 12 is a side view of a flared fixation element, in accordance with an embodiment.
FIG. 13 is a sectional view of a distal portion of a biostimulator having an electrically active fixation element, in accordance with an embodiment.
FIG. 14 is a side view of a fixation element having an insulated section, in accordance with an embodiment.
FIG. 15 is a detail sectional view of a fixation element having an insulated section, in accordance with an embodiment.

### DETAILED DESCRIPTION

Embodiments describe a biostimulator, such as a leadless pacemaker, having a stiffened pacing element. As described below, the biostimulator can be used to perform deep septal pacing of a heart. The biostimulator may, however, be used in other applications, such as deep brain stimulation. Thus, reference to the biostimulator as being a cardiac pacemaker for deep septal pacing is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator for deep septal pacing includes a radiopaque marker. The radiopaque marker aligns with a distal end of a fixation element mount on which a fixation element is mounted. The radiopaque marker can therefore provide visual feedback under fluoroscopy to indicate a position of the fixation element mount relative to a septal wall. Accordingly, using the visual feedback, a physician can confirm that the leadless cardiac pacemaker is accurately affixed to the septal wall.

In an aspect, a biostimulator for deep septal pacing includes a fixation element having a flared section. The flared section can capture septal tissue to anchor the biostimulator in a septal wall. Accordingly, the fixation element can maximize tissue capture to mitigate a risk of dislodgment.

In an aspect, a biostimulator includes a fixation element that is electrically coupled to circuitry. Accordingly, the fixation element can both anchor the biostimulator in a septal wall and deliver pacing impulses into the septal wall to a target tissue.

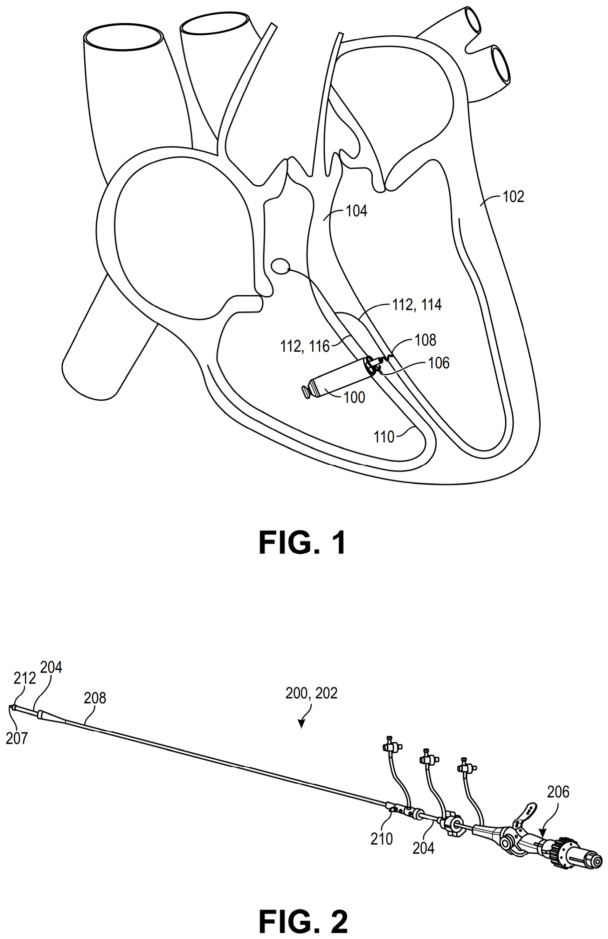

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulators 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to a septum 104 of the heart 102. More particularly, the biostimulator 100 can be delivered to the septum 104, and one or more elements, such as a fixation element 106 and/or a pacing element 108 can pierce a septal wall 110 of the septum 104 to engage and anchor the biostimulator 100 to the tissue. In a particular embodiment, the biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems. In some implementations of transport systems, a leadless pacemaker is attached or connected to a distal end of a catheter and advanced intravenously into or out of the heart 102. The transport system can include features to engage the leadless pacemaker to allow fixation of the leadless pacemaker to tissue. For example, in implementations where the leadless pacemaker includes an active engaging mechanism, such as a fixation element 106, the transport system can include a docking cap or key at a distal end of the catheter, and the docking cap or key may be configured to engage the leadless pacemaker and apply torque to screw the active engaging mechanism into or out of the tissue. In other implementations, the transport system includes clips designed to match the shape of a feature on the leadless pacemaker and apply torque to screw the active engaging mechanism into or out of the tissue.

When the biostimulator 100 is delivered to and screwed into the septum 104 of the heart 102, the pacing element 108 and/or the fixation element 106 may be positioned for deep septal pacing at respective bundle branches 112 in the septum 104. For example, an active electrode of the pacing element 108 can be positioned at the left bundle branch 114 in the septum 104. Similarly, the fixation element 106 can be positioned at or proximal to the right bundle branch 116 in the septum 104. Optionally, one of the elements may be at a bundle branch and the other element may not be at a bundle branch.

Referring to FIG. 2, a perspective view of a biostimulator system is shown in accordance with an embodiment. A biostimulator system 200 includes the biostimulator 100 (not shown) mounted on a biostimulator transport system 202. As described above, the biostimulator 100 can be delivered to and retrieved from a patient anatomy using the biostimulator transport system 202. In some implementations, the biostimulator transport system 202 is a delivery system for delivering the leadless pacemaker to the target tissue. In some implementations, the biostimulator transport system 202 is a retrieval system for retrieving the leadless pacemaker from the target tissue. The biostimulator transport system 202 can include a release mechanism to retain any of the biostimulators described below in an unreleased state and to transition into a released state to release the biostimulators into the target anatomy.

The biostimulator transport system 202 can include an elongated catheter 204 extending distally from a handle 206 to a distal catheter end 207. The elongated catheter 204 can be a deflectable catheter, and an operator can use the handle 206 to steer the distal catheter end 207 in the patient. In an embodiment, the biostimulator transport system 202 includes a guide catheter 208 mounted on the elongated catheter 204. The guide catheter 208 can be slidably disposed on the elongated catheter 204 such that a distal portion of the guide catheter 208 can slide distally over the distal catheter end 207 of the elongated catheter 204 and/or the biostimulator 100, which may be mounted on the distal catheter end (not shown). Similarly, the biostimulator transport system 202 can include an introducer hub assembly 210 mounted on the guide catheter 208. The introducer hub assembly 210 can be slidably disposed on the guide catheter 208 such that a distal portion of the introducer hub assembly 210 can slide distally over the distal catheter end 207 of the elongated catheter 204 and/or the distal portion of the guide catheter 208. More particularly, the introducer hub assembly 210 can be inserted into an access sheath to gain access to the patient vasculature, and after access is established, the distal portion of the guide catheter 208 and/or the distal catheter end 207 of the elongated catheter 204 can be advanced through the access sheath into the patient.

The distal catheter end 207 of the elongated catheter 204 may be selectively connectable to the biostimulator 100. More particularly, the biostimulator 100 can be mounted on the distal catheter end 207 of the elongated catheter 204. The biostimulator 100 can be protected by a protective sheath of the distal portion of the guide catheter 208 during delivery and/or retrieval of the biostimulator 100 from the patient. Accordingly, the biostimulator 100 can be advanced into the patient along with the distal catheter end 207.

The leadless pacemaker system can be used to implant one or more biostimulators 100 within an atrium and/or a ventricle of a heart 102 of the patient. Implantation of each biostimulator 100 may be achieved, in part, by endocardial insertion of the biostimulators 100. For example, the elongated catheter 204 of the leadless pacemaker system can include a torque shaft coupled to a docking cap 212. The docking cap 212 can have a docking cavity to receive an attachment feature of the biostimulator 100. The torque shaft can be torqueable and rotation of the torque shaft can rotate the docking cap 212, which can impart rotation to the attachment feature. Accordingly, torque can be transmitted through the torque shaft to rotate the biostimulator 100 in a first direction, e.g., clockwise. Rotating the biostimulator 100 when a fixation element 106 is in contact with the heart tissue can cause the fixation element to screw into the heart tissue and affix the biostimulator 100 to the heart tissue. Similarly, removal and retrieval of the biostimulators 100 may be accomplished endocardially. For example, the torque shaft of the elongated catheter 204 can be rotated in a second direction, e.g., counterclockwise, to transmit torque through the docking cap 212 to the attachment feature to disengage the biostimulator 100 from the heart tissue.

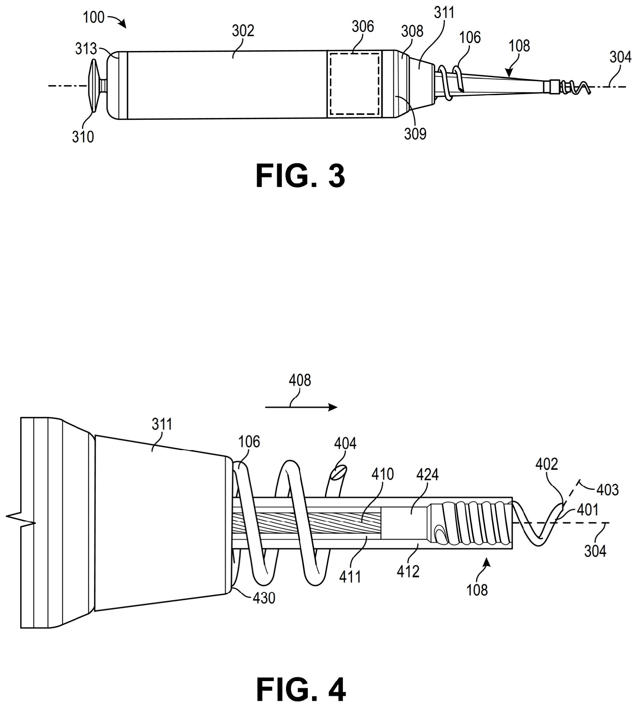

Referring to FIG. 3, a side view of a biostimulator having a pacing element is shown in accordance with an embodiment. The biostimulator 100 can be a leadless cardiac pacemaker that can perform cardiac pacing and that has many of the advantages of conventional cardiac pacemakers while extending performance, functionality, and operating characteristics. The biostimulator 100 can have two or more electrodes, e.g., a portion of a pacing element 108 that acts as an active electrode and/or a portion of the fixation element 106 or a housing 302 that acts as an active electrode. The electrodes can deliver pacing pulses to bundle branches 112 within the septum 104 of the heart 102 to perform deep septal pacing, and optionally, can sense electrical activity from the muscle. The electrodes may also communicate bidirectionally with at least one other device within or outside the body.

In an embodiment, the biostimulator 100 includes the housing 302 having a longitudinal axis 304. The housing 302 can contain a primary battery to provide power for pacing, sensing, and communication, which may include, for example, bidirectional communication. The housing 302 can optionally contain an electronics compartment 306 (shown by hidden lines) to hold circuitry adapted for different functionality. For example, the electronics compartment 306 can contain circuits for sensing cardiac activity from the electrodes, circuits for receiving information from at least one other device via the electrodes, circuits for generating pacing pulses for delivery to tissue via the electrodes, or other circuitry. The electronics compartment 306 may contain circuits for transmitting information to at least one other device via the electrodes and can optionally contain circuits for monitoring device health. The circuit of the biostimulator 100 can control these operations in a predetermined manner. The biostimulator 100 can perform leadless pacing without a pulse generator located in the pectoral region or abdomen and/or without an electrode-lead separate from the pulse generator. The biostimulator 100 may also lack a communication coil or antenna, and may not have the substantial battery power required for transmitted communication through a communication coil or antenna.

Leadless pacemakers or other leadless biostimulators 100 can be fixed to an intracardial implant site, e.g., at the septal wall 110, by one or more actively engaging mechanisms or fixation mechanisms, such as a screw or helical member that screws into the myocardium. In an embodiment, the biostimulator 100 includes the fixation element 106 coupled to the housing 302. The fixation element 106 can include a helical fixation element and/or several tines. More particularly, the biostimulator 100 can include a header assembly having a flange 308 of the housing 302. The flange 308 can be coupled to a distal housing end 309 of a body of the housing 302. The fixation element 106 can be coupled to and extend distal to the flange 308. For example, a fixation element mount 311 can be mounted on the housing, e.g., on the flange 308, and the fixation element 106 can be mounted on the fixation element mount 311. The fixation element 106 can extend about the longitudinal axis 304. For example, in the case of a helical fixation element, the element can spiral about the longitudinal axis to a distal fixation tip. In the case of a fixation element 106 having several tines, the tines can be arranged about the longitudinal axis and extend to respective distal tips. Accordingly, when the biostimulator 100 is delivered to the target tissue, the distal tip(s) of the fixation element 106 can pierce the tissue and the housing 302 can be rotated or pushed to affix the fixation element 106 to the target tissue.

In an embodiment, the biostimulator 100 includes an attachment feature 310. The attachment feature 310 can be mounted on a proximal housing end 313 of the housing 302. More particularly, the attachment feature 310 can be mounted on an opposite end of the housing 302 from the fixation element 106 and the pacing element 108, which as described above, can be coupled to the distal housing end 309 of the housing 302. The attachment feature 310 can facilitate precise delivery or retrieval of the biostimulator 100. For example, the attachment feature 310 can be formed from a rigid material to allow a transport system to engage the attachment feature 310 and transmit torque and/or axial loads to the attachment feature to plunge the fixation element 106 or the pacing element 108 into the target tissue.

Referring to FIG. 4, an end view of a biostimulator having a pacing element is shown in accordance with an embodiment. The biostimulator 100 can include the pacing element 108 coupled to the housing 302. The pacing element 108 can be coaxially arranged with the fixation element 106 about the longitudinal axis 304. More particularly, the pacing element 108 can extend along, e.g., axially along or helically about, the longitudinal axis 304 at a location that is radially inward from the fixation element 106.

The pacing element 108 can extend distally to a tip electrode 401. The tip electrode 401 can extend along a spiral axis 403. More particularly, the spiral axis 403 can revolve about the longitudinal axis 304, and the tip electrode 401 can extend along the spiral axis 403 to a distal pacing point 402. The distal pacing point 402 may be distal to the distal fixation tip 404. The pacing element 108 can include a helical element to screw into a target tissue or a conical element to pierce into the target tissue. Accordingly, when the fixation element 106 screws into or otherwise engages the target tissue, the pacing element 108 can also engage the tissue, and the housing 302 can be advanced and/or rotated to cause the distal pacing point 402 of the pacing element 108 to pierce the tissue and anchor the biostimulator 100.

One or more of the fixation element 106 or the pacing element 108 can be an active electrode, and can electrically communicate with the circuitry contained in the electronics compartment 306. Accordingly, the anchored element(s) can electrically communicate with the tissue and can transmit electrical pulses between the tissue and the circuitry of the biostimulator 100. To facilitate electrical function of the fixation element 106 and/or the pacing element 108, the element(s) may be coated in titanium nitride to reduce a polarization value of the electrode(s). By way of example, the titanium nitride coating may be in a range of 5 to 50 microns, e.g., 13 microns.

The biostimulator 100 can include a fixation element mount assembly that includes the fixation element 106 mounted on the fixation element mount 311. The fixation element mount 311 can be non-conductive. For example, the fixation element mount 311 can be formed from polyether ether ketone (PEEK). The fixation element 106 can include MP35N wire having a wire diameter of 0.4-0.6 mm, e.g., 0.5 mm. The wire may be formed from a metal, such as stainless steel or a nickel cobalt alloy. The wire can be wrapped into several turns to form a helical fixation element. The turns can be screwed into the septum 104 to anchor the biostimulator 100 within the heart 102.

The fixation element mount 311 can have a distal mount end 430. The distal mount end 430 may, during an operation, be pressed into the septal wall. More particularly, engagement of the distal mount end 430 with the septal wall can be indicative of a well-seated biostimulator 100, which is optimally placed for effective pacing. When the distal mount end 430 is pressed against the septal wall, the tip electrode 401 can engage the target tissue to deliver pacing pulses.

In an embodiment, the pacing element 108, which is coupled to the housing 302, extends in a distal direction 408 to the distal pacing point 402. More particularly, the pacing element 108 can include a core assembly that extends between the electronics compartment 306 and the distal pacing point 402. The core assembly can include one or more structural members to carry electrical pulses, such as a flexible conductor 410. The flexible conductor 410 can extend in the distal direction 408, e.g., along the longitudinal axis 304, through the fixation element mount 311 and the fixation element 106 to the distal pacing point 402 distal to the distal fixation tip 404.

In an embodiment, the pacing element 108 of the biostimulator 100 includes an insulation sleeve 412 surrounding, e.g., encapsulating, the flexible conductor 410. More particularly, the insulation sleeve 412 can cover and/or surround the flexible conductor 410. The insulation sleeve 412 can insulate a length of the pacing element 108. For example, the electrically conductive component(s) of the flexible conductor 410 can be encapsulated by the insulation sleeve 412 over its length, including over a portion of the flexible conductor 410 distal to the fixation element 106.

In an embodiment, the pacing element 108 includes an intermediate insulation liner 411 between the flexible conductor 410 and the insulation sleeve 412. The insulation liner 411 may, for example, include a polymer tube separating at least a portion of the flexible conductor 410 from the insulation sleeve 412. The insulation liner 411 can be formed from an insulative polymer such as a synthetic fluoropolymer, e.g., polytetrafluoroethylene (PTFE). Accordingly, pacing impulses delivered through the flexible conductor 412 can be insulated from a surrounding tissue within which the insulation sleeve 412 is located by the insulation liner 411 and/or insulation sleeve 412. The pacing impulses can therefore be delivered through the flexible conductor 410 to the pacing tip to pace the target tissue.

The pacing element 108 can extend distal to the fixation element mount 311 by a length sufficient for the tip electrode 401 of the pacing element 108 to access the target region, e.g., the left bundle branch 114. The left bundle branch 114 may be at a depth of 10-25 mm from the septal wall 110, typically. Accordingly, the pacing element 108 can have a length of 7-20 mm, e.g., 12 mm or 16 mm, from a distal end of the fixation element mount 311 to the distal pacing point 402. Accordingly, the pacing element 108 can provide a means of pacing a target tissue that is distal to tissue being engaged by the fixation element 106. For example, the pacing element 108 can pace the left bundle branch 114 when the fixation element 106 is engaged near a surface of the septal wall 110 and/or near the right bundle branch 116.

The helical tip electrode 401 may be mounted on a tip support 424. The tip support 424 can include a base, which may be bonded to or crimped on to the flexible conductor 410. A boss can extend distal to the base. The boss may be inserted into an inner channel of the distal pacing element, e.g., the helical tip electrode 401. The helical tip electrode 401 may be secured to the tip support 424, e.g., by welding the helical electrode to the base or the boss. Accordingly, the distal tip electrode 401 can be secured to the flexible conductor 410 to receive and relay pacing impulses to the target tissue at the distal pacing point 402.

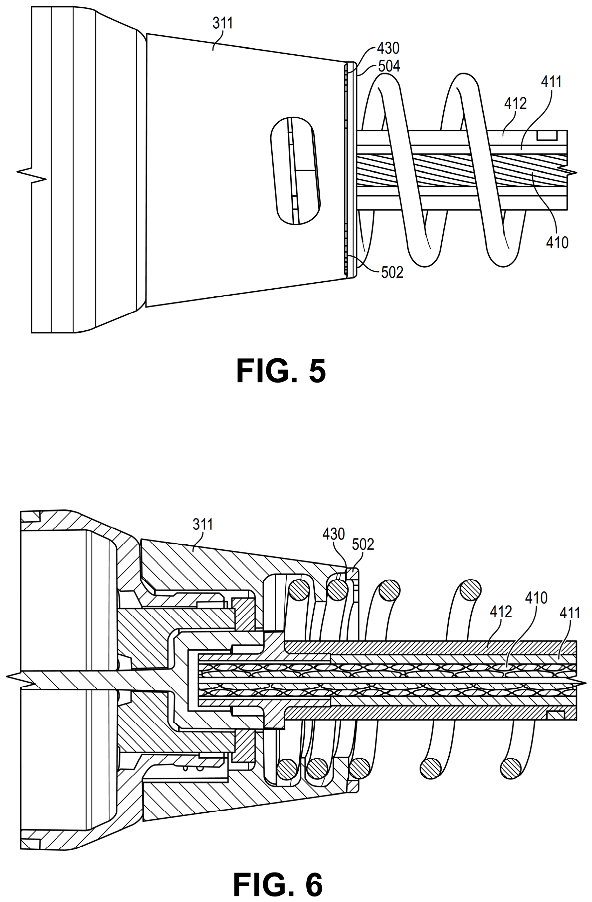

As described below with respect to FIGS. 5-10, the biostimulator 100 can include radiopaque markers to enhance fixation by improving visualization under fluoroscopy. More particularly, radiopaque markers indicating a position of the fixation element mount 311 can provide visual feedback to determine whether the fixation element mount 311 is seated at the septal wall 110 and the fixation element 106 is anchored in the septal tissue. Similarly, radiopaque markers indicating a position of the tip electrode 401 can provide visual feedback to determine whether the tip electrode 401 is positioned in the target tissue.

The radiopaque indicator associated with the fixation element mount 311 can allow physicians to distinguish where a distal surface of a non-radiopaque fixation element mount 311 is located under fluoroscopy during an implantation procedure. When used in conjunction with a radiopaque contrast, the physicians will have the ability to see where the septal tissue surface is in comparison to the distal surface of the fixation element mount 311. The physicians can therefore receive vital feedback in knowing when to stop torquing the biostimulator 100 into the septal tissue. As described below, the radiopaque indicator can include one or more of an individual ring component, medical adhesive doped with a radiopaque material, a separate radiopaque component installed flush with a top surface of the fixation element mount 311, or a fixation element mount 311 formed from PEEK doped with a radiopaque material.

Referring to FIG. 5, a side view of a distal portion of a biostimulator having a radiopaque marker is shown in accordance with an embodiment. The biostimulator 100 can include a radiopaque marker 502 to indicate a location of the device under fluoroscopy. In an embodiment, the radiopaque marker 502 can indicate the location of the distal mount end 430. Accordingly, the radiopaque marker 502 can have a distal marker end 504 that is longitudinally aligned with the distal mount end 430 of the fixation element mount 311. The principle of indicating the distal mount end 430 under fluoroscopy is applied to various embodiments, as described below. Accordingly, the illustrated concept of FIG. 5 is representative of and applies to the various radiopaque marker embodiments described below.

Referring to FIG. 6, a sectional view of a distal portion of a biostimulator having a radiopaque marker is shown in accordance with an embodiment. The radiopaque marker 502 can be mounted on the fixation element mount 311 at the distal mount end 430. For example, the radiopaque marker 502 can include a radiopaque marker ring that is installed on an outside of a distal edge of the fixation element mount 311. More particularly, the ring may be attached to the distal edge. Attachment may be performed by snapping the ring onto the edge or press fitting the ring into a groove or onto an exterior surface of the edge. Alternatively, the ring may joined to the edge, e.g., via an adhesive or thermal weld. Locking features, such as threads, may similarly be used to connect the radiopaque marker 502 to the fixation element mount 311. In any case, the radiopaque marker 502 can be fixed to the distal mount end 430 of the fixation element mount 311.

The radiopaque marker 502 may be embedded in the fixation element mount 311 at the distal mount end 430. For example, a ring of radiopaque material may be embedded, e.g., overmolded, in the fixation element mount 311 during manufacturing. Similar to mounting the marker on the distal edge of the mount, embedding the marker can indicate the location of the distal mount end 430 under fluoroscopy.

The radiopaque marker 502 may be formed in various shapes using various materials. For example, the marker may be a ring, as described above. The ring may be a complete ring, e.g., circular, or a partial ring, e.g., c-shaped. The biostimulator 100 may include a single marker or several markers, e.g., radiopaque pucks, located around the lip of the fixation element mount 311. In an embodiment, the radiopaque marker 502 is formed from a radiopaque, biocompatible material. For example, the marker may be formed from MP35N, however, other materials such as tantalum may also be used to form the marker.

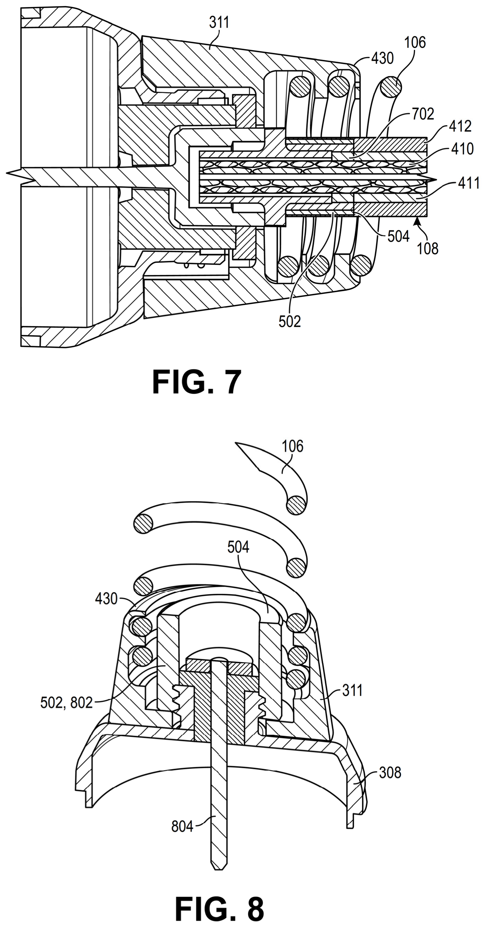

Referring to FIG. 7, a sectional view of a distal portion of a biostimulator having a radiopaque marker is shown in accordance with an embodiment. The radiopaque marker 502 may be coupled to the pacing element 108. The radiopaque marker 502 can include a ring or tubular element installed within the extended electrode subassembly of the biostimulator 100. For example, the marker may be embedded in the insulation sleeve 412 of the pacing element 108. Embedding the marker may include reflowing the electrode subassembly around the radiopaque marker 502 during manufacturing.

Rather than embedding the marker, the radiopaque marker 502 may be mounted. More particularly, the radiopaque marker 502 can be mounted on the insulation sleeve 412 of the pacing element 108. Mounting can include slipping the marker into a lumen of the insulation sleeve 412. Alternatively, the marker can be placed around an outer surface of the insulation sleeve 412. For example, the radiopaque marker 502 can have a central channel 702, e.g., a channel of a tubular marker, and the pacing element 108 can be placed into the marker to extend through the central channel 702. The tubular marker may then be crimped or adhered to the pacing element 108 to affix and secure the marker relative to the pacing element 108. In an embodiment, the marker is locked in place via medical adhesive back-fill within an interior of the fixation element mount 311.

The radiopaque marker 502 can have a tubular shape, as shown in FIG. 7. Alternatively, the marker have another shape that allows it to be located on, in, or within the pacing element 108. For example, the marker can include one or more longitudinal rods, a coil, a braided tube, etc. In any case, the marker can be internal, external, or embedded in the insulation of the pacing element 108.

As described above, the radiopaque marker 502 can align with the distal mount end 430 to indicate a location at which the fixation element mount 311 presses against the septal wall 110. A distal end of the radiopaque marker 502, e.g., a distal edge of the tubular marker, may therefore be located at a same longitudinal location as the distal mount end 430. The adjacency and alignment of the distal ends of the mount and the marker allow the distal mount end 430 location to be visualized under fluoroscopy despite the fixation element mount 311 not having substantial radiopacity.

Referring to FIG. 8, a sectional view of a distal portion of a biostimulator having a radiopaque marker is shown in accordance with an embodiment. The radiopaque marker 502 can retain the fixation element mount 311 against the housing 302. More particularly, the radiopaque marker 502 can hold the fixation element mount 311 relative to the flange 308 of the housing 302. In an embodiment, the radiopaque marker 502 includes a marker nut 802. The marker nut 802 can act as a fastener to engage the flange 308. For example, the marker nut 802 can have an internal thread that screws onto an external thread of the flange 308. The marker nut 802 can be threaded onto the flange 308 of the housing 302 to capture the fixation element mount 311 between the flange 308 and the marker nut 802.

The marker nut 802 can be radiopaque. For example, the marker nut 802 can include a radiopaque doped polymer. The polymer may be used in an injection molding process to form the nut with or without the internal threads. More particularly, the internal threads may be formed when the polymer nut is screwed onto the metal flange threads. The polymer nut can electrically insulate the fixation element 106 from electrically conductive components, such as the flange 308 or an electrode pin 804 of a feedthrough. Accordingly, the marker nut 802 can perform several functions, including: insulating electrically active components from each other, securing the fixation element mount 311 to the housing 302, and indicating a location of the distal mount end 430.

The marker nut 802 may be formed from any of several radiopaque materials, including MP35N LT, platinum iridium, or Ti G2 alloys. The marker nut 802 can include the distal marker end 504, e.g., at a distal end of a tubular section of the marker nut 802. The distal marker end 504 can align with the distal mount end 430. By making the distal ends of the marker and the mount flush with each other, the location at which the fixation element mount 311 engages tissue can be viewed under fluoroscopy.

Optionally, a space between the radiopaque marker 502 and the surrounding structures, such as the fixation element mount 311 and/or the fixation element 106, may be filled to secure or further insulate the components of the header assembly. For example, a medical adhesive backfill may optionally be used to fill a cavity around the marker nut 802, between the marker nut 802 and the fixation element 106. As described below, such filler materials may further be used to enhance visualization of the fixation element mount 311.

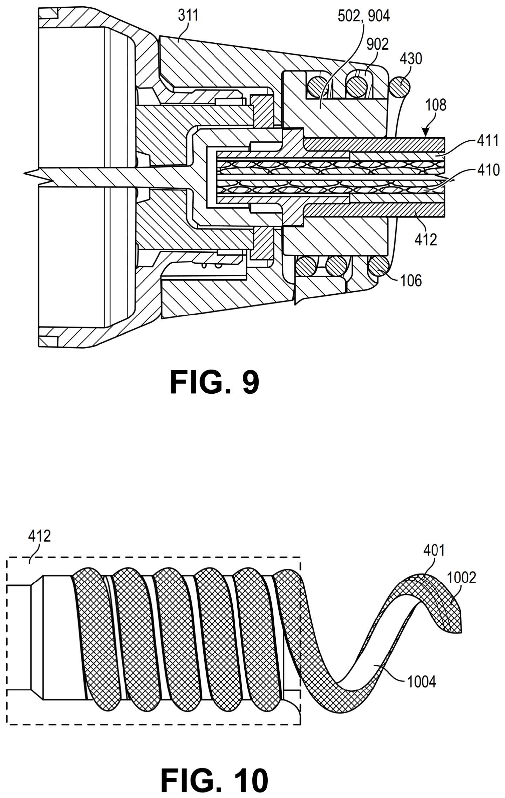

Referring to FIG. 9, a sectional view of a distal portion of a biostimulator having a radiopaque marker is shown in accordance with an embodiment. The radiopaque marker 502 can include a filler material disposed within the space, e.g., a cavity 902, channel, or opening, contained by the fixation element mount 311. The biostimulator 100 can include a cavity 902 between the pacing element 108 and the fixation element mount 311. The cavity 902 can include a space between threads of the fixation element mount 311, an annular space between the fixation element 106 and the insulation sleeve 412, etc. In an embodiment, a radiopaque doped filler 904 is disposed in the cavity 902. More particularly, the radiopaque doped filler 904 can fill all or a portion of the cavity 902.

The radiopaque doped filler 904 can fill the cavity 902 longitudinally to the distal mount end 430. For example, a distal end of the filler can be aligned with, at a same longitudinal distance as, the distal mount end 430. Alignment may be approximate. For example, the filler may have a meniscus that causes the distal ends to be adjacent to each other longitudinally. In any case, the distal ends can be aligned to allow the radiopaque doped filler 904 to sufficiently approximate the longitudinal location of the distal mount end 430 under fluoroscopy.

The radiopaque doped filler 904 can include a medical adhesive doped with a radiopaque material that is backfilled into the open region within the fixation element mount 311. Alternatively, the filler can include a doped epoxy. The filler may be formed from an insulative material. In any case, the filler can allow the fixation element mount 311 to be visible under fluoroscopy to allow a physician to visualize engagement between the fixation element mount 311 and the septal tissue.

In an embodiment, the fixation element mount 311 may be formed from a radiopaque material. For example, the fixation element mount 311 can include a radiopaque doped polymer. The entire body of the fixation element mount 311 can be formed from such material. A minority of the fixation element mount 311 may be doped, by volume. For example, the radiopaque doped polymer can include PEEK loaded with less than 15% by volume of a radiopaque material. The radiopaque material can include barium sulfate or titanium dioxide. In either case, the doping of the mount body may be 9-13% by volume. Advantageously, maintaining doping levels in the described ranges can provide the visualization benefit sought, while avoiding embrittlement of the fixation element mount 311, which can occur at higher doping levels.

Conjunctively with the fixation element mount 311 visualization aid described above, the biostimulator 100 can include a spray-coat polymer for the tip electrode 401. More particularly, the spray-coat polymer can be doped with a radiopaque material and applied as a partial insulation on the tip electrode helix. The coating can allow a physician to have a more precise visualization of the actual pacing tip reaching the target tissue, e.g., at the bundle branch 112.

Referring to FIG. 10, a side view of a tip electrode of a biostimulator is shown in accordance with an embodiment. At least a portion of the tip electrode 401 can be coated with a radiopaque coating 1002. The radiopaque coating 1002 may include, for example, a radiopaque material that is miscible, biocompatible, and non-interfering of a function of the tip electrode 401 or any other coating constituents. Other coating materials can include materials to increase or decrease overall impedance, e.g., a low polarization material, to increase battery life and longevity of the device. More particularly, the radiopaque coating 1002 can be supplementary or complementary to the tip electrode function by improving visibility of the tip electrode 401 during implantation.

The radiopaque coating 1002 can be spray coated onto an outer surface of the tip electrode 401. During the coating process, a portion of the tip electrode 401 may be masked. For example, a plug may be located within an interior of the helical tip electrode 401 to mask an inner radius of the helical surface. The plug can be removed after the coating process. The radiopaque material may therefore be disposed, e.g., sprayed, on an outer radius of the tip electrode 401, and the inner radius can remain a bare, uncoated surface 1004. The outer radius may, however, have a coating that can, among other things, enhance visibility of the tip electrode 401. A physician can therefore visualize whether the pacing member is in the bundle branch area under fluoroscopy.

In addition to enhancing fixation by improving visualization, the biostimulator 100 can enhance fixation via mechanical or electrical function of the fixation element 106. A geometry of the fixation element 106 can enhance fixation of the biostimulator 100 to the septal tissue. More particularly, the fixation element 106 can be shaped to reliably and securely capture tissue to anchor the biostimulator 100 in the septum 104. Furthermore, the fixation element 106 may be dual purpose and used as an electrically active electrode. More particularly, as described below, the fixation element 106 may be used solely to secure the biostimulator 100 to the surface of the septal wall 110, or may have an additional function of acting as an electrically active electrode.

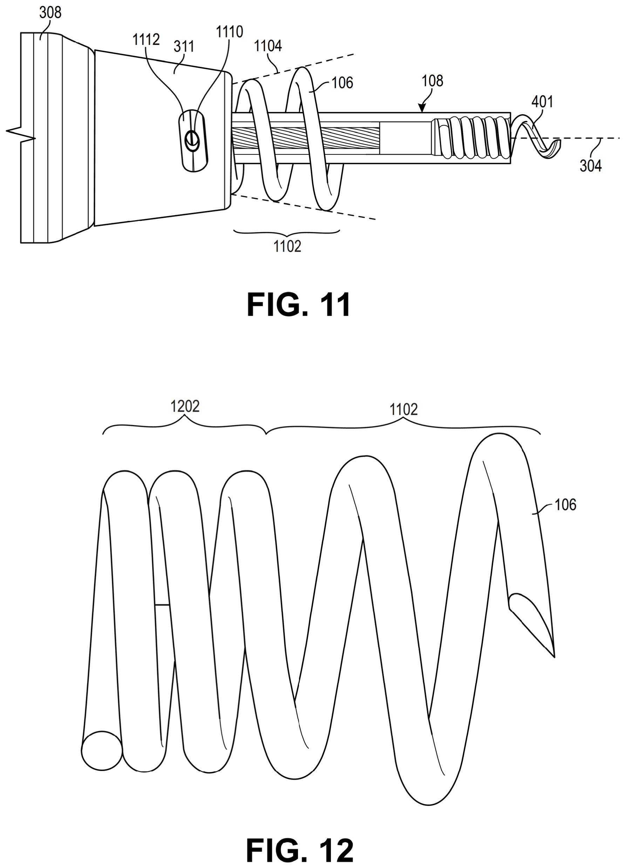

Referring to FIG. 11, a side view of a distal portion of a biostimulator having a flared fixation element is shown in accordance with an embodiment. The fixation element 106 can include an electrically inactive helix that is secured within the fixation element mount 311 and extends distally out of the mount toward the tip electrode 401. The fixation element 106 can extend helically about the longitudinal axis 304 as the element revolves around the pacing element 108. More particularly, the electrically inactive fixation element 106 can revolve about the electrically active inner extended pacing electrode that protrudes from the fixation element mount 311. The pacing electrode can burrow into the septal tissue to access the left bundle branch 114 during implantation. The fixation element 106 can anchor the biostimulator 100 at the septal wall 110 during such pacing.

In an embodiment, the fixation element 106 includes a flared section 1102 having an outer diameter 1104 that increases in a distal direction 408. The flared section 1102 can be slightly tapered or flared outward as the helix extends distally out of the fixation element mount 311. Advantageously, the increasing diameter of the fixation element 106 can create an enlarging space between the fixation element 106 and the pacing element 108. Accordingly, the tapered shape allows for additional septal tissue to be captured between the pacing element 108 and an inner surface of the fixation element 106. The additional tissue capture can enhance fixation by increasing an overall strength of connection to the tissue. More particularly, the tapered fixation element 106 can increase an amount of tissue squeezed and a contact surface area between the fixation element 106 and the captured tissue, which can increase a dislodgement torque required to back the fixation element 106 out of the tissue. The biostimulator 100 can be more reliably anchored to the tissue, as a result.

The fixation element 106 can be secured within the fixation element mount 311 via a weld 1110. For example, the fixation element mount 311 can include a weld window 1112, e.g., an opening extending through a sidewall of the mount, and the weld 1110 may be formed through the window. The weld 1110 may be aligned with the weld window 1112 to lock the fixation element 106 to the helix mount. The weld 1110 can include a fusion of several turns of the fixation element helix, or a spot weld of a single turn. In either case, the weld 1110 can bulge radially outward into the weld window 1112. Movement of the fixation element 106 can be impeded by the bulge. For example, the bulge can contact an edge of the window when the fixation element 106 moves, and limit additional movement of the fixation element 106. The weld 1110 may therefore prevent the helix from backing out of the mount.

In an additional or alternative mechanism of action, to impede movement of the fixation element 106, the weld 1110 can pool material together between adjacent features of the fixation element 106. For example, the fixation element 106 can include a helix having several turns, and the weld 1110 can pool material between adjacent turns. The pooled material can reduce a likelihood that the fixation element 106 will back out of the fixation element mount 311. The helix can be screwed into an internal thread of the fixation element mount 311 prior to forming the weld 1110. The internal thread can be interrupted by the weld window 1112. Accordingly, when the weld is formed and the helix is rotated within the internal thread, the weld 1110 can move toward an edge of the window. The weld spot will engage the internal thread as the helix is rotated, and the weld will act as a natural stopping point that prevents separation of the successive turns by the internal thread. More particularly, the internal thread will engage the weld and further rotation of the helix will be impeded, preventing back out of the fixation element 106 from the fixation element mount 311.

Referring to FIG. 12, a side view of a flared fixation element is shown in accordance with an embodiment. The fixation element 106 having the flared section 1102 can include a coil section 1202 that does not taper. More particularly, the coil section 1202 can include a length of coil having a constant outer diameter. The coil section 1202 can extend proximally from the flared section 1102. The constant outer diameter can conform to an inner diameter of the fixation element mount 311. Accordingly, the coil section 1202 can be loaded into the cavity 902 of the mount. When the coil section 1202 is loaded, the turns of the section can be viewed and welded through the weld window 1112 to lock the fixation element 106 in place.

The variation in outer diameter 1104 over the length of the fixation element 106 can have a corresponding variation in structural stiffness. For example, a stiffness of the flared section 1102 of the fixation element 106 can decrease in the distal direction 408. The decreasing stiffness may be inversely proportional to the outer diameter 1104 of the helix. As the outer diameter 1104 increases, the longitudinal stiffness, e.g., an amount that the helix will extend or compress in response to an axial load, can decrease. Accordingly, the fixation element 106 can both capture additional tissue and reduce a risk of tissue trauma during implantation.

The variation in outer diameter may also provide a visual feedback to a user. More particularly, the overall shape and transition point of the coil section 1202 to the flared section 1102 may indicate a location of the distal mount end 430 of the fixation element mount 311 under fluoroscopy. Such indication may be inherent in a shape change of the fixation element 106, and require no supplemental marker at the transition point, such as a separate marker, doped epoxy, or doped mount material, as described with respect to alternate embodiments herein. More particularly, the fixation element 106 may be formed from a material having sufficient radiopacity to be accurately visualized under fluoroscopy. Thus, the coil section 1202 may not taper, and the fixation element 106 may begin to taper only at the distal mount end 430 of the fixation element mount 311. Accordingly, a point where the taper begins, which can be longitudinally aligned seen and inferred to be the location of the distal mount end 430, under visualization.

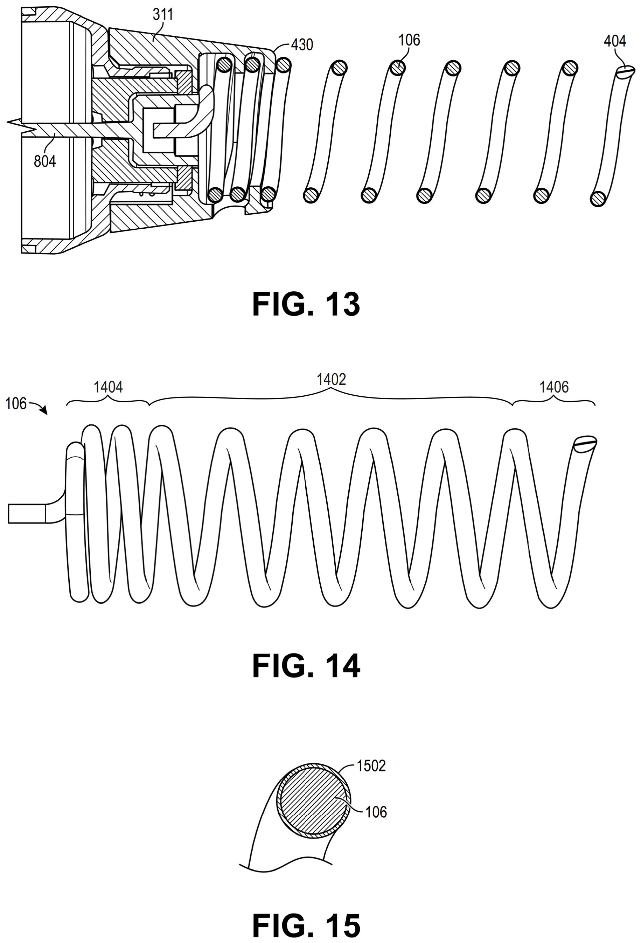

Referring to FIG. 13, a sectional view of a distal portion of a biostimulator having an electrically active fixation element is shown in accordance with an embodiment. The fixation element 106 can be electrically active. In an embodiment, the fixation element 106 is electrically coupled to the circuitry contained in the electronics compartment 306. For example, a proximal end of the fixation element 106 can be electrically connected to the electrode pin 804 of the feedthrough, which carries pacing impulses distally from the circuitry. The connection may be via a weld or a conductive potting that attached the fixation element end to an electrode cup at a distal end of the electrode pin 804.

The active fixation element 106 helix can be an only helix of the device. More particularly, the extended electrode pacing element 108 described above may be omitted when using the electrically active fixation element 106. The electrically active fixation helix can be secured within the fixation element mount 311, e.g., using the weld 1110 through the weld window 1112, and can extend distally to the distal fixation tip 404. A length of the fixation element 106 may be sufficient to place the distal fixation tip 404 at the target tissue, e.g., the bundle branch region, when the distal mount end 430 of the fixation element mount 311 is pressed against the septal tissue.

Referring to FIG. 14, a side view of a fixation element having an insulated section is shown in accordance with an embodiment. During implantation, the fixation element 106 helix can be torqued into the septal tissue to a depth that allows for electrical activation of the target tissue. The required length can cause an increase in exposed surface area, of electrically active pacing surface, to the tissue. To account for the substantial increase in overall size and surface area of the electrode when compared to the tip electrode 401 described above, a selective insulation can be used to cover a portion of the fixation element 106. More particularly, the fixation element 106 can include an insulated section 1402 between a proximal exposed section 1404 and a distal exposed section 1406. The selective insulation may be applied as a parylene coating. More particularly, parylene can be deposited on the insulated section 1402 using a vapor deposition process.

In an embodiment, the partially insulated fixation element 106 can have a pitch that allows the distal fixation tip 404 to reach the target tissue with a predetermined number of rotations of the biostimulator 100. For example, a pitch of the fixation element 106 helix can be set to provide for the tip to reach the target tissue a set number of rotations, e.g., 1.5 turns, after the tip enters the septal tissue.

Referring to FIG. 15, a detail sectional view of a fixation element having an insulated section is shown in accordance with an embodiment. The insulated section 1402 can include an insulative coating 1502 covering the electrically conductive surface of the underlying fixation element 106. The insulation can limit the exposed conductive area and impedance of the fixation element 106.

The proximal end of the fixation element 106, e.g., the proximal exposed section 1404, can be located within the fixation element mount 311. The exposed section may therefore be insulated partially by the surrounding fixation element mount 311. Furthermore, a backfill material may be disposed around the proximal exposed section 1404, e.g., filling the cavity 902 of the mount, to insulate the entire proximal portion of the fixation element 106. Accordingly, electrical current can terminate at the distal tip of the pacing element 108, to allow electricity to be conserved and directed to the target tissue. Ultimately, through coating and insulating the electrically active fixation helix, the pacing capture threshold, impedance, and polarization of the electrically active fixation element 106 can be maintained.

In the foregoing specification, the invention has been described with reference to specific exemplary embodiments thereof. It will be evident that various modifications may be made thereto without departing from the broader spirit and scope of the invention as set forth in the following claims. The specification and drawings are, accordingly, to be regarded in an illustrative sense rather than a restrictive sense.

## Claims

1. A biostimulator (100), comprising:
a housing (302) having a longitudinal axis (304) and containing an electronics compartment (306);
a pacing element (108) coupled to the housing (302), wherein the pacing element (108) includes a flexible conductor (410) extending along the longitudinal axis (304);
a fixation element mount (311) mounted on the housing (302), wherein the fixation element mount (311) includes a distal mount end (430);
a fixation element (106) mounted on the fixation element mount (311), wherein the fixation element (106) extends about the longitudinal axis (304); and
a radiopaque marker (502) having a distal marker end (504) longitudinally aligned with the distal mount end (430) of the fixation element mount (311).

2. The biostimulator of claim 1, wherein the radiopaque marker (502) is mounted on the fixation element mount (311) at the distal mount end (430).

3. The biostimulator of claim 1, wherein the radiopaque marker (502) is embedded in the fixation element mount (311) at the distal mount end (430).

4. The biostimulator of claim 1, wherein the radiopaque marker (502) is coupled to the pacing element (108).

5. The biostimulator of claim 4, wherein the pacing element (108) extends through a central channel (702) of the radiopaque marker (502).

6. The biostimulator of claim 4 or 5, wherein the radiopaque marker (502) is embedded in an insulation sleeve (412).

7. The biostimulator of claim 4 or 5, wherein the radiopaque marker (502) is mounted on an insulation sleeve (412).

8. The biostimulator of claim 1, wherein the radiopaque marker (502) includes a marker nut (802), and wherein the marker nut (802) is threaded onto a flange (308) of the housing (302) to capture the fixation element mount (311) between the flange (308) and the marker nut (802).

9. The biostimulator of claim 8, wherein the marker nut (802) includes a radiopaque doped polymer.

10. The biostimulator of claim 1, wherein the radiopaque marker (502) includes a radiopaque doped filler in a cavity (902) between the pacing element (108) and the fixation element mount (311).

11. The biostimulator of claim 1, wherein the fixation element mount (106) includes a radiopaque doped polymer.

12. The biostimulator of claim 11, wherein the radiopaque doped polymer includes polyether ether ketone loaded with less than 15% by volume of a radiopaque material.

13. The biostimulator of claim 1, wherein the pacing element (108) includes a tip electrode (401) extending along a spiral axis (403) about the longitudinal axis (304), and wherein at a portion of the tip electrode (401) is coated with a radiopaque coating (1002).

14. The biostimulator of any one of claims 1 to 13, wherein the fixation element (106) extends helically about the longitudinal axis (304), and wherein the fixation element (106) includes a flared section (1102) having an outer diameter (1104) that increases in a distal direction.

15. The biostimulator of claim 1, wherein the housing (302) contains circuitry in the electronics compartment (306), wherein the fixation element (106) is electrically coupled to the circuitry, and wherein the fixation element (106) includes an insulative coating (1502) between a proximal exposed section (1404) and a distal exposed section (1406).
